# EUROPEAN PATENT SPECIFICATION

(11) **EP 4 616 828 B1**
(45) Date of publication and mention of the grant of the patent: **11.11.2026**
(21) Application number: 24214264.4
(22) Date of filing: 20.11.2024
(51) Int. Cl.: A61F 2/24

(54) **ANNULOPLASTY RING WITH DELAYED RELEASE**
ANNULOPLASTIERING MIT VERZÖGERTER FREISETZUNG
ANNEAU D'ANNULOPLASTIE À LIBÉRATION RETARDÉE

(30) Priority: 11.03.2024 US 202463563648 P
(43) Date of publication of application: 17.09.2025
(73) Proprietor: St. Jude Medical, Cardiology Division, Inc., St. Paul, MN 55117 (US)
(72) Inventor: Von Oepen, Randolf, Aptos, 95003 (US)
(74) Representative: Gill Jennings & Every LLP

(56) References cited:
- EP-A1- 3 628 274
- WO-A1-2020/186106
- US-A1- 2016 256 276
- US-A1- 2019 038 411

## Description

### Background of the Disclosure

Heart valve disease is a significant cause of morbidity and mortality. One treatment for this disease is valve replacement. Native atrioventricular valves (*i.e.,* the tricuspid valve and the mitral valve) typically have a larger size and/or diameter compared to the native aortic valve and the native pulmonary valve. Among the native atrioventricular valves, a regurgitant tricuspid valve typically has a larger size and/or diameter than a regurgitant mitral valve. For example, for patients with severe tricuspid valve regurgitation, the diameter of the tricuspid valve may range from about 40 mm to about 66 mm, although these numbers are merely exemplary.

Initial human trials of transfemoral replacement of the tricuspid (*i.e.,* right atrioventricular) valve have shown promising results. However, one of the issues associated with right side heart failure (*e.g.*, tricuspid valve regurgitation) is that the annulus of the tricuspid valve is often significantly dilated. As noted above, the tricuspid valve annulus in these patients may have sizes up to 60 mm in diameter or more. If a transcatheter replacement valve is implanted in such a large annulus, it is possible that a significant amount of foreign material (*e.g*., metal, fabric, or tissue structures of the prosthetic heart valve) will be positioned in a low-flow area near the tricuspid valve. This may create significant risk of blood clotting (*e.g.,* thrombosis), which can result in an embolism *(e.g.,* a pulmonary embolism for the right heart or a stroke for the left heart). Furthermore the frame of the prosthetic valve may tend to inhibit desirable remodeling of the right ventricle, even if the prosthetic heart valve successfully treats the tricuspid valve regurgitation.

Another treatment for heart valve disease is annuloplasty, in which the annulus of the heart valve is resized and/or reshaped to help the native valve leaflets more completely coapt to reduce regurgitation or backflow through the native heart valve.

Annuloplasty procedures are typically performed using a ring or a band that is fixed to the annulus of the native heart valve. In some examples, the annuloplasty ring is a rigid band that is sutured (or otherwise coupled) directly to the native heart valve annulus during an open heart surgery while the patient is on cardiopulmonary bypass. In these types of procedures, the rigidity of the ring forces the native annulus to resize and/or reshape due to the rigidity of the ring. However, open heart surgery is highly invasive and it would be preferable to achieve similar or the same results as surgical annuloplasty rings without needing to perform open heart surgery. Percutaneous annuloplasty rings have been attempted, but have not gained significant traction because the procedures are typically highly complex and very time consuming, with mixed clinical success.

It would therefore be desirable to be able to treat the tricuspid valve with an annuloplasty procedure to avoid the need for a prosthetic heart valve, while also avoiding the problems of the known annuloplasty procedures. EP3628274A1 describes a mitral annulus repair device configured to be implanted within the subject's left atrium, such that no portion of the device extends through the subject's mitral valve.
WO2020/186106A1 describes cardiac valve repair devices with annuloplasty features. US2019/038411A1 describes a heart valve annulus repair device having a tissue engaging member and a plurality of anchors.

### Brief Summary of the Disclosure

The invention provides an annuloplasty ring system in accordance with appended claim 1. One of the side apices of each of the plurality of diamond-shaped cells may be connected to one of the side apices of an adjacent one of the plurality of diamond-shaped cells. The corresponding one of the plurality of diamond-shaped cells may include a top aperture formed in the top apex and a bottom aperture formed in the bottom apex, and the corresponding one of the plurality of biodegradable connectors may have a first end knotted at the top aperture and a second end knotted at the bottom aperture. The plurality of biodegradable connectors may be biodegradable sutures. The frame may be configured to transition from the initial condition to the final condition upon degradation of the biodegradable sutures. The biodegradable sutures may be configured to maintain the frame in the initial condition for more than about one month following implantation of the annuloplasty ring into a patient. The biodegradable sutures may be configured to maintain the frame in the initial condition for more than about two months following implantation of the annuloplasty ring into a patient. The biodegradable sutures may be configured to maintain the frame in the initial condition for about three months following implantation of the annuloplasty ring into a patient.

Each of the plurality of cells may have an initial height and an initial width in the initial condition of the frame, and a final height and a final width in the final condition of the frame. The initial height may be smaller than the final height, and the final width may be smaller than the initial width. The skirt may be formed of fabric. The skirt may include a first skirt portion and a second skirt portion, the first skirt portion being coupled to the bottom surface of the frame, the second skirt portion being coupled to a top surface of the frame so that the frame is sandwiched between the first skirt portion and the second skirt portion. The skirt may be laminated over the frame to encapsulate the frame therein. The frame may include a plurality of hooks, tines, or barbs extending away from the bottom surface of the frame.

The annuloplasty ring system may further include adhesives on one or both of (i) the bottom surface of the frame and (ii) the skirt. The annuloplasty ring system may further include a sizing device, and the sizing device may include a plurality of expandable arms. Each of the plurality of expandable arms may have a first end a free end remote from the first end, the first end of each of the plurality of expandable arms being coupled together at a first connection point. The sizing device may include a plurality of actuator arms, each of the actuator arms having a first end coupled to a corresponding one of the plurality of expandable arms, the second end of each of the plurality of actuator arms being coupled together at a second connection point. The sizing device may include a pull wire extending through a coil member, the pull wire being coupled to the second connection point such that pulling the pull wire expands the plurality of actuator arms radially outwardly, and expansion of the plurality of actuator arms radially outwardly causes the plurality of expandable arms to expand radially outwardly.

### Brief Description of the Drawings

Fig. 1 is a highly schematic cross-section of a heart illustrating four native heart valves.
Fig. 2A is a top view of an example of an annuloplasty ring.
Fig. 2B is a top view of an example of a frame of the annuloplasty ring of Fig. 2A in an initial assembled state.
Fig. 2C is a top view of the example of the frame of Fig. 2B in a set shape.
Fig. 2D is a schematic illustration showing a change in size of a cell of the frame of Fig. 2B over time.
Fig. 2E is a schematic top view showing a change in configuration of the annuloplasty ring of Fig. 2A over time.
Fig. 2F is a highly schematic cross-section of the annuloplasty ring of Fig. 2A implanted into a tricuspid valve annulus showing the tricuspid valve annulus changing over time.
Fig. 3A is a schematic cross-sectional view of a shaping device in contact with the annulus of the native tricuspid valve.
Fig. 3B is a cut-away top view of the configuration shown in Fig. 3A.
Fig. 3C is a schematic view of the annuloplasty ring of Fig. 2A having been implanted over the shaping device of Fig. 3A.

### Detailed Description of the Disclosure

This disclosure is generally directed to devices and methods for reducing the size of a tricuspid valve annulus (*e.g.* tricuspid valve annuloplasty) to reduce or eliminate tricuspid valve regurgitation. As is described in greater detail below, at least some examples described herein rely on an annuloplasty ring or similar device that is implanted into the tricuspid valve annulus in a transcatheter procedure, but which does not reduce the size of the tricuspid valve annulus until after the device is partially or entirely healed (and therefore strongly secured) within the tricuspid valve annulus, which may be a delay of a few weeks or up to three months or more.

Although the disclosure herein is described in connection with devices and methods to perform tricuspid valve annuloplasty, the concepts described herein may also be applicable to other heart valves, including the mitral valve. However, even if the devices and methods described herein are applicable to both mitral valves and tricuspid valves, they may be particularly suited to the tricuspid valve for a number of reasons. For example, the annulus expansion at a regurgitant tricuspid valve is frequently significantly greater than that found at a regurgitant mitral valve. Further, the right heart is typically less muscular than the left heart, and therefore the tricuspid valve annulus typically requires less force to reduce the size of the annulus compared to the mitral valve. Further, in patients with regurgitant tricuspid valves, the entire right side of the heart can be greatly expanded, and a full replacement valve may not allow remodeling of the right heart. An annuloplasty ring of the type described herein, on the other hand, may continue to reduce the annulus diameter after it has fully healed into the tissue. Still further, patients with tricuspid valve regurgitation are typically more suited to a delayed elimination of regurgitation compared to patients with mitral valve regurgitation, at least in part because mitral valve regurgitation can create more significant symptoms compared to tricuspid valve regurgitation.

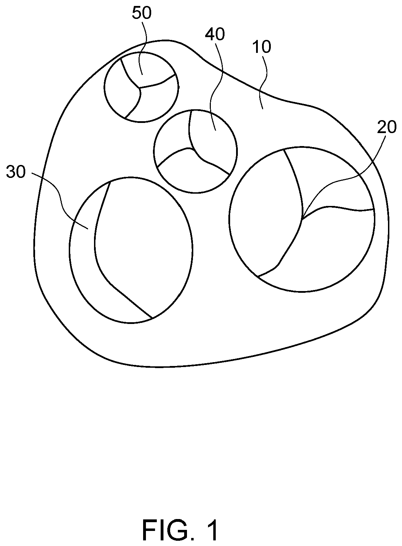

Fig. 1 is a highly schematic cross-section of a heart 10 illustrating four native heart valves, including the tricuspid valve 20, the mitral valve 30, the aortic valve 40, and the pulmonary valve 50. As can be seen in Fig. 1, the tricuspid valve 20 and the mitral valve 30 are significantly larger than the counterpart aortic valve 40 and pulmonary valve 50.

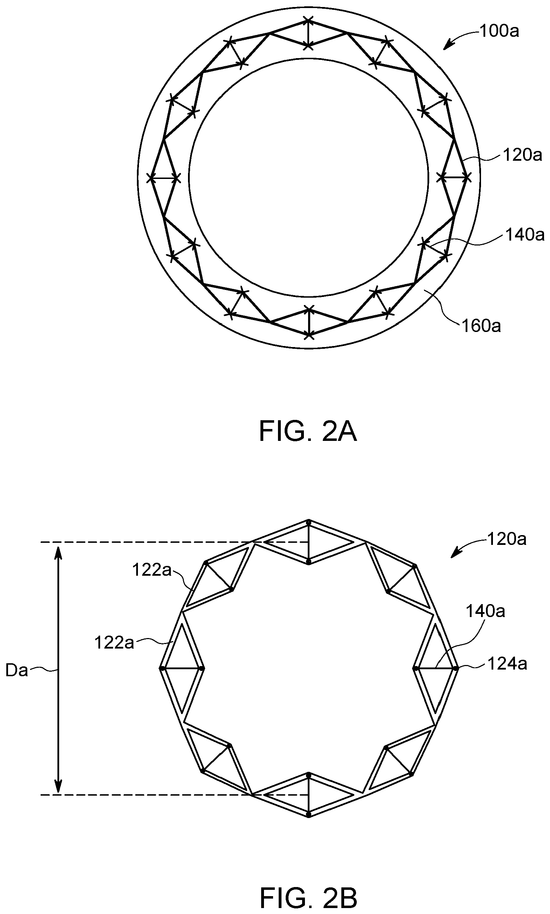

Fig. 2A is a schematic top view of an example of an annuloplasty ring 100a. The part numbers provided in connection with Figs. 2A-F ending in an "a" indicate that the component is an in initial assembled state *(e.g.,* just prior to or just after implantation), whereas part numbers ending in a "b" indicate that the component is in a final steady state or a pre-assembly state. Part numbers lacking the "a" or "b" designation are intended to refer to the relevant component generally. In the illustrated example, the annuloplasty ring 100a includes a frame 120a, a plurality of connectors 140a, and a skirt 160a. In some examples, the frame 120a is formed of a shape-memory material, such as a nickel-titanium metal alloy such as nitinol. In some examples, the frame 120a may be formed by laser cutting a flat sheet of nitinol, although other mechanisms for manufacturing the frame 120a are suitable.

Fig. 2B is a top view of the frame 120a with connectors 140a assembled to the frame 120a, but with the skirt 160a omitted for clarity of illustration. The shape of the assembled frame 120a include a plurality of diamond-shaped cells attached end-to-end in a continuous circumferential pattern. Although Fig. 2A illustrates a total of twelve diamond-shaped cells, and Fig. 2B illustrates a total of eight diamond-shaped cells, it should be understood that other numbers of cells (including odd numbers of numbers greater than or smaller than eight or twelve) may be suitable. Each diamond-shaped cell may include four struts 122 that form opposing top and bottom apices and opposing side apices. In the illustrated embodiment, the top apex and bottom apex of each cell includes a hole or aperture 124a formed therein. As will be described in greater detail below, the apertures 124a may be used to receive connectors 140a, such as sutures (including biodegradable or bioabsorbable sutures) to temporarily maintain the frame 120a in the initial assembled condition 120a. However, in other embodiments, the apertures 124a may be omitted and instead the connectors may be coupled directly to struts 122a of the cells (or to other features of the cells).

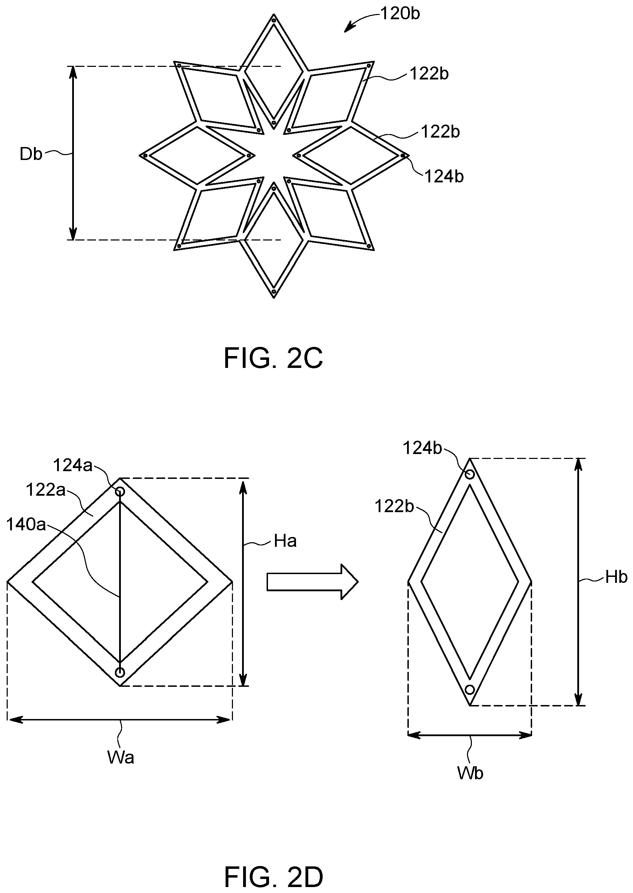

Fig. 2C is a top view of the frame 120b after having been formed and shape-set. The view of Fig. 2C may also correspond to the shape of the frame 120b after it has been implanted and returned mostly or fully to its set shape. As with Fig. 2B, the skirt is omitted from the view of Fig. 2C for ease of illustration. However, the connectors 140a are not illustrated in Fig. 2C because, in the configuration of Fig. 2C, the connectors 140a have either not yet been assembled to the frame 120b (in order to transition the frame to the configuration of Fig. 2B), or otherwise the connectors 140a have already degraded and the frame 120a has transitioned to the condition of frame 120b. The main difference between frames 120a and frame 120b is that the cells of the frame 120b are in their set-shape in which the top and bottom apices of the cells are farther apart (compared to the condition of frame 120a) while the side apices of the cells are closer together (compared to the condition of frame 120a). It should be understood that, in both conditions of frame 120a, 120b, the top surface *(e.g.,* the surface visible in Fig. 2A) and the bottom surface *(e.g.,* the surface facing "into the page" in the view of Fig. 2A) may be substantially flat.

Referring to Figs. 2A-2C, after forming the frame 120 *(e.g.,* via laser cutting a flat sheet or tube of nitinol), it may be shape-set (*e.g*., via heat treatment) to have a relatively small outer diameter Db, as shown in Fig. 2C. After shape-setting the frame 120b to have the small diameter Db, it may be temporarily transitioned to a larger diameter frame 120a. For example, as shown in Fig. 2B, the top and bottom apex of each diamond-shaped cell may be drawn toward each other, which results in a reduction in the height of each cell, but an increase in the circumferential width of each cell, and thus an increase in the diameter Da of the frame 120a. While the frame 120a is in the increased diameter condition, connectors 140a may be coupled to each cell of the frame 120a to maintain each cell at the decreased height, despite the fact that the frame 120b is shape-set to a smaller diameter. For example, a plurality of connectors 140a may each be tied to a top apex of a cell via one aperture 124a, and to a bottom apex of the respective cell via another aperture 124a. The connectors 140a are preferably secured under tension so that the connectors 140a prevent the frame 120a from transitioning back to the set shape in which the height of each cell increases while the width of each cell decreases. Each connector 140a is preferably formed of as biodegradable suture, thread, or wire that will break down over time within the body following implantation. It should be understood that the diameter Da of frame 120a and the diameter Db of frame 120b generally refer to the diameter of a circle that passes through the side apices of each cell. However, in other example the relevant diameter may be the outer diameter of the frame or the inner diameter of the frame. In practice, the diameter that may matter the most is the diameter defined by the points of connection of the frame to the annulus tissue, as should become clear below.

Referring to Fig. 2A, the frame 120a may be generally annular in the initial condition, and one or more skirts or cuffs 160a may be coupled to the frame 120a. In one example, one skirt 160a is coupled to a bottom surface of the frame 120a so that, upon implantation, the skirt 160a will directly contact native tissue of the heart valve annulus (or otherwise tissue adjacent to the annulus, such as tissue adjacent the annulus facing into the right atrium). The skirt 160a may be formed of a fabric, or any other material that promotes tissue ingrowth. In some embodiments, a top skirt may also be provided so that the frame 120a is sandwiched between top and bottom skirts 160a. In other embodiments, two skirts 160a may be laminated together over the frame 120a to encapsulate the frame 120a therein. Whether one or more skirts 160a are provided, the skirt(s) 160a may in some examples be coated or impregnated or otherwise modified with a material (*e.g*., fibronectin) to promote tissue ingrowth (*e*.*g*., by causing a foreign body reaction).

Fig. 2D is a schematic illustration showing a change in size of a cell of the frame 120 over time. For example the frame 120 may be shape-set so that the struts 122b of each cell have a relatively large height Hb and relatively small circumferential width Wb (shown on the right of Fig. 2D). When initially forming the annuloplasty ring 100a, the apices of each cell may be brought together to decrease the height and increase the width, and the connector 140a may be knotted or otherwise fixed to the apertures 124a to temporarily maintain the cells in this shorter *(e.g.,* smaller height Ha), wider *(e.g.,* larger width Wa) condition (shown on the left of Fig. 2D). Following implantation, as described in greater detail below, the connector 140a may degrade over time until it is no longer able to maintain tension between the ends of the connector 140a, causing the cell to revert (or begin to revert) to its set shape. In other words, during initial assembly, the cells transition opposite the direction of the arrow in Fig. 2D, but over time after implantation, each cell will tend to transition with the direction of the arrow in Fig. 2D after the connector 140a degrades.

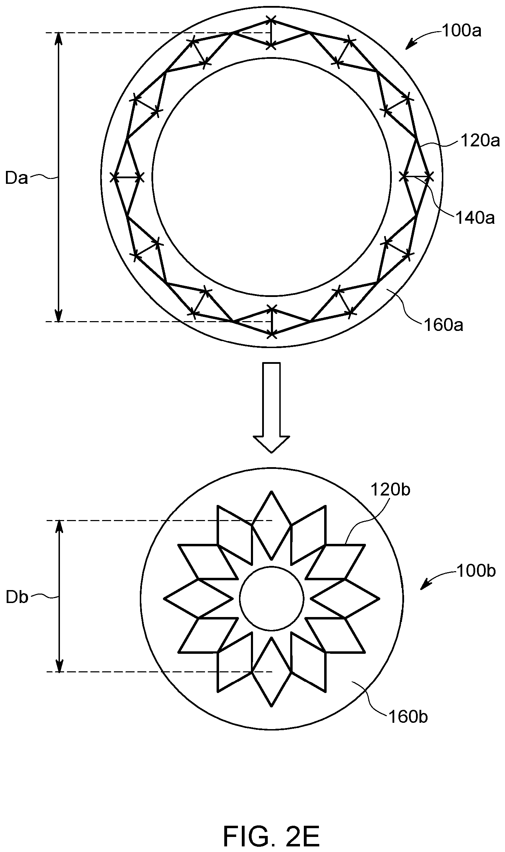

Fig. 2E is a schematic top view showing a change in configuration of the annuloplasty ring 100a from its initial condition upon implant to its final configuration after the annuloplasty ring 100b reverts partly or fully back to its set shape. As can be seen, the annuloplasty ring 100a may be implanted while it has a temporarily large diameter Da. Over time, for example from two or three weeks to three or more months, tissue ingrowth will begin to occur on skirt 160a as the healing process of the patient's body progresses. As the tissue ingrowth occurs, the skirt 160a, and thus the frame 120a, become more and more securely fixed to the native valve annulus. Preferably, the connectors 140a are selected so that they do not significantly degrade until significant tissue ingrowth has occurred and the frame 120a and skirt 160a are very securely fixed to the native valve annulus. Once the healing process has completed (or at least progressed significantly), the connectors 140a may degrade to the point where they can no longer hold tension, and thus can no longer maintain the cells of the frame 120a in the temporarily wider and shorter condition. As each connector 140a degrades, the corresponding cell will start to transition back toward its set shape (as shown in Fig. 2D), causing the annuloplasty ring 100b and frame 120b to transition back toward the initial set shape with the smaller diameter Db (as best shown in Fig. 2E). As this transition occurs, the tissue ingrowth has already strongly secured the annuloplasty ring within the native annulus such that the annuloplasty ring 100b pulls the native annulus to a smaller diameter as it transitions.

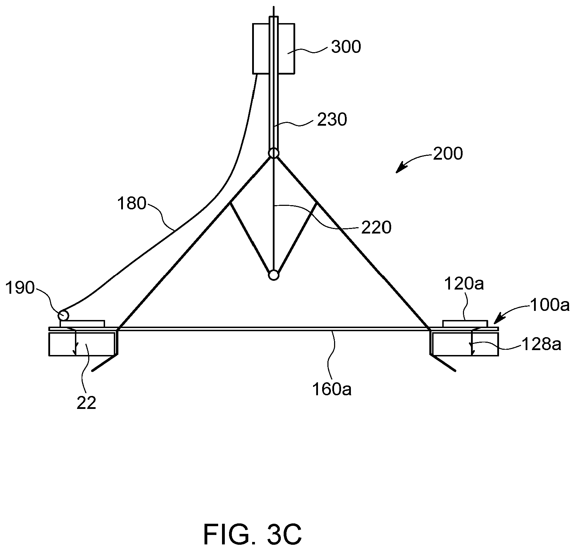

Although not shown in Figs. 2A-2E, the initial securement of the annuloplasty ring 100a to the annulus (prior to tissue ingrowth providing enhanced fixation) may be achieved by one or more mechanisms. In some examples, adhesives such as tissue glue may be applied to the bottom surface of the frame 120a and/or skirt 160a to assist with the initial fixation. In some examples, including those shown in connection with Fig. 3C below, tines, barbs, or other anchors may be provided on the frame 120a, including either integrally formed with the frame 120a or formed separately and later connected. In further examples, a secondary procedure may be performed to drive separate anchors (*e.g*., barbs, "T"-shaped anchors, corkscrew anchors, *etc*.) through the frame 120a and into the tissue immediately after placing the annuloplasty ring 100a in contact with the tissue. It should be understood that one or more of these options, or other suitable options for initial anchoring, may be combined.

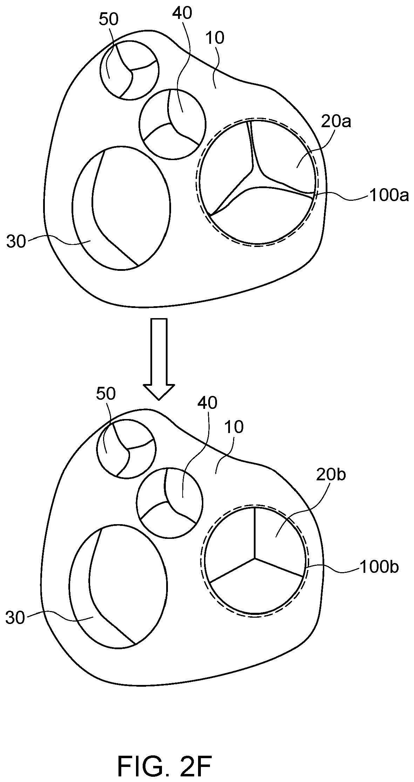

Fig. 2F is a highly schematic illustration showing annuloplasty ring 100a implanted within the annulus of the native tricuspid valve 20a. In Fig. 2F, for simplicity of illustration, the annuloplasty ring 100a, 100b is shown only as a dashed circle. As can be seen in Fig. 2F, the top of the figure represents an initial condition in which the annuloplasty ring 100a has the temporarily large diameter, and the native tricuspid valve 20a has a relatively large diameter and the leaflets of the tricuspid valve are not efficiently closing. Over time, after the annuloplasty ring has been "locked" into the annulus via tissue ingrowth, the connectors 140a degrade to allow the annuloplasty ring 100b to transition back to its smaller diameter size. As shown in the bottom view of Fig. 2F, after the annuloplasty ring 100b has transitioned partially or fully back to its original set shape, it pulls the annulus of the tricuspid valve 20b inwardly to reduce its size, enhancing the coaptation of the leaflets of the tricuspid valve 20b, reducing or eliminating regurgitation.

In some examples, the connectors 140a may be selected to degrade over a desired timepoint, for example after about 2 or 3 weeks, after about 1 month, after about 2 months, or about after 3 months. The degradation time may be fine tuned in some examples by the type and the size of the connector 140a. In some examples, the connectors 140a may be formed of biodegradable polymers, including for example polyglycolic acid ("PGA"), polylactic acid ("PLA"), polycaprolactone ("PCL"), or copolymers thereof. In some examples, each connector 140a may be substantially identical to each other connector. However, in other examples, different connectors 140a may be used to stagger the amount of time the connectors take to degrade. For example, some connectors 140a may be configured to degrade after 4 weeks, some connectors 140a may be configured to degrade after 6 weeks, some connectors 140a may be configured to degrade after 8 weeks, some connectors 140a may be configured to degrade after 10 weeks, and some connectors 140a may be configured to degrade after 12 weeks. With this type of configuration, individual cells of the frame 120a will begin to transition back to their set shape in a staggered timeline, which may allow for a more gradual decrease in diameter of the frame 120b, compared to all of the connectors 140a degrading at about the same time which may result in a large amount of force over a small amount of time. It should be understood that the particular combination of connectors 140a (if different connectors are used) may be any suitable combination to allow for a more gradual diameter shift, whether there are only two different types of connectors 140a or more than two different types of connectors 140a.

As should be understood from the above description, one of the goals of the annuloplasty ring 100 is to force the tricuspid valve annulus back into or near its original shape prior to its dilation as part of the heart disease process. Because the expansion or dilation of the tricuspid valve annulus typically does not occur over a very short period, it may be beneficial to have a correspondingly slow reduction in the size of the tricuspid valve annulus via the transition of the annuloplasty ring from the initial condition 100a back toward the shape-set condition 100b. Nitinol is one desirable option for forming frame 120 because, after the connectors 140a degrade, the nitinol frame 120 will continuously try to reach its original set shape. The frame 120 of annuloplasty ring 100 will at all times be in an equilibrium of forces after implantation. When all the connectors 140a have degraded, the frame 120a will reduce it diameter until an equilibrium of force has been achieved (which accounts for opposing forces applied by the native tissue on the annuloplasty ring). However, if the frame 120a has not yet achieved its final diameter Db, it will continue to shrink over time. Nitinol is also flexible, and a flexible frame 120 may be advantageous as the heart muscle is in constant motion and the flexibility of the frame 120 will not unduly hinder necessary motion of the heart muscle, particularly compared to stiff, surgically implanted annuloplasty rings or surgically implanted prosthetic heart valves.

Although the illustrations of Fig. 1 and Fig. 7 illustrate the tricuspid valve 20 as a circle, in practice, the annulus of the tricuspid valve 20, particularly in patients that have a disease state necessitating treatment, is typically not a circle. Because the annuloplasty ring 100a may form a circle, a mismatch in shape between the annuloplasty ring 100a and the annulus of the tricuspid valve 20 may exist prior to performing the annuloplasty procedure. This may be problematic, because in order for tissue ingrowth to secure the annuloplasty ring 100a to the tissue, contact between the tissue and the annuloplasty ring 100a is typically needed. In other words, tissue ingrowth will not occur, or at least is less likely to occur, at locations where the annuloplasty ring 100a is not in contact with tissue. Further, because the particular size and shape of the annulus will not be identical among all patients, a single shape of annuloplasty ring 100a will not likely be achieve good contact with the tissue of all patients without an additional feature.

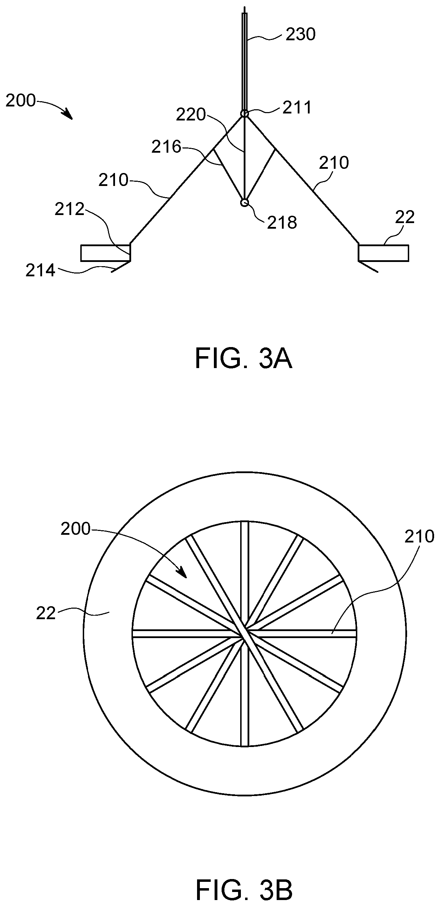

Fig. 3A is a schematic cross-sectional view of a shaping device 200 in contact with the annulus 22 of the native tricuspid valve 20. The shaping device 200 may include a plurality of expanding arms 210. The expanding arms 210 may each extend from a first end to a second free end. The first ends of each of the expanding arms 210 may come together (*e.g.,* be connected) at a central location, such as a ring or connector 211, and the free ends of each expanding arm may include contours, such as hooks or ramped extensions, configured to hook around the annulus 22. In the illustrated example, each free end of each expanding arm 210 may include a straight segment 212 configured to press against an inner surface of the annulus 22, and an angled terminal segment 214 configured to hook around the bottom or outflow end of the annulus. In the illustrated embodiment, a total of twelve expanding arms 210 (shown in Fig. 3B) are included at substantially equal intervals around the circumference of the shaping device 200. However, in other embodiments, more or fewer expanding arms 210 may be provided. In some embodiments, at least three expanding arms 210 are included to assist with self-centering of the shaping device 200. Further, in some embodiments, the expanding arms 210 (if formed of shape-memory material such as nitinol) may be shape set *(e.g.,* via heat treatment) to the expanded condition similar to that shown in Fig. 3A.

Still referring to Fig. 3A, an actuator arm 216 may be attached to each expanding arm 210. In some embodiments, the actuator arms 216 are integral with the expanding arms 210, and may be attached via a hinge such as a living hinge. In other embodiments, the actuator arms 216 are formed separately and attached to the expanding arms 210. Each actuator arm 216 may include a first end coupled to a corresponding expanding arm 210, and a second end attached to the other actuator arms 216. With this configuration, while the expanding arms 210 generally extend radially outward from a central longitudinal axis (when in the expanded condition), the actuator arms 216 generally extend from the extending arms 210 toward the central longitudinal axis. At the point of connection between the plurality of actuator arms 216, a ring 218 or similar structure may be provided. In some examples, the expanding arms 210 and actuator arms 216 are formed from the same material, such as stainless steel, nitinol, or other metals or metal alloys. In other examples, the expanding arms 210 and actuator arms 216 may be formed of different materials.

In some embodiments, an actuator or pull wire 220 may be provided as part of the shaping device 200. In some examples, the pull wire 220 may be formed of a cable, which may be made from metal or metal alloys, including stainless steel. The pull wire 220 may have a terminal end that is coupled to the ring 218, or in some examples may be looped through the ring 218. In either example, an end of the pull wire 220 is preferably accessible outside the body by a user so that the pull wire 220 may be manually pulled (or pulled via an actuator on a handle of a delivery device that is connected to the pull wire 220). In use, as the shaping device 220 is delivered into or near the annulus 22, the expanding arms 210 and actuator arms 216 may extend parallel or near parallel to each other and to a central longitudinal axis of the shaping device 200 and/or annulus 22. With the expanding arms 210 positioned interior to the annulus 22, the user may retract pull wire 220. Proximal force on pull wire 220 is transmitted to ring 218, tending to pull ring proximally, causing the actuator arms 216 to "open" away from each other and thus causing the expanding arms 210 to "open" away from each other. As the expanding arms 210 move radially outwardly, they eventually contact the inner surface of the annulus 22, for example with the straight segments 212 directly contacting the annulus 22 and the angled terminal segments 214 hooking around the outflow portion of the annulus 22, helping to keep the expanding arms 210 in contact with the annulus 22. As additional proximally-directed force is applied to the pull wire 220, the expanding arms 210 will continue to expand, forcing the annulus 22 to reshape into a generally circular shape, as best shown in Fig. 3B. Further, the expanding arms 210 may form a general cone shape at this stage, with the smaller diameter end of the cone shape positioned above the annulus 22 and the larger diameter of the cone shape positioned at or adjacent to the annulus 22. However, even if the annulus 22 is not reshaped to a perfect circle, as will become clear below, the shaping device 200 may be used to deliver and/or deploy the annuloplasty ring 100a *(e.g.,* using the ramped surface defined by the cone shape of the expanding arms 210), such that both the annuloplasty ring 100a and the annulus 22 take the same shape defined by the shaping device 200.

It should be understood that the shaping device 200 may be delivered to the patient through a transvascular catheter *(e.g.,* a catheter extending in the antegrade direction thorough the femoral vein, through the inferior vena cava, into the right atrium, and extending into or near the tricuspid valve) while the expanding arms 210 and actuator arms 216 are in a collapsed condition. In some examples, a coil 230 may be coupled to and/or in contact with the ring or connector 211 and/or the connected ends of the expanding arms 210. In this example, the pull wire 220 may extend through a central axis defined by coil 230. If the coil 230 is included, when the pull wire 220 is pulled proximally, the coil 230 may help provide a counterforce so that the expanding arms 210 and actuator arms 216 expand outwardly instead of simply also pulling proximally.

Fig. 3C is a schematic view of the annuloplasty ring 100a having been implanted over shaping device 200 and onto the annulus 22. In an exemplary method of use, a delivery catheter 300 may be advanced into the right atrium, which may for example be a steerable delivery catheter 300. As the delivery catheter 300 is delivered to or adjacent to the annulus 22, the shaping device 200 may be in a collapsed condition within the delivery catheter 300. In other examples, the delivery catheter 300 may be delivered first, and then the shaping device 200 may be advanced through the delivery catheter 300. The shaping device 200 may be pushed distally out of the delivery catheter 300 (or the delivery catheter 300 may be withdrawn relative to the shaping device 200) so that the shaping device 200 exits the catheter 300. Upon exiting the catheter 300, the shaping device 200 may begin to expand. In some examples, the expansion may be at least in part due to the expanding arms 210 tending to self-expand. In some examples, the expanding arms 210 may be forced to open by pulling pull wire 220 proximally. In some examples, the expansion may be due to both. Regardless of the particular method to achieve expansion, the expansion arms 210 may be expanded into contact with the interior surface of the annulus 22 *(e.g.,* as shown in Figs. 3A-3C), and further expanded to force the annulus 22 into a generally circular shape (although in some embodiments this further expansion is not necessary, or results in a non-circular shape of the annulus 22). The steps described above in some examples will result in a configuration similar to that shown in Figs. 2A-2B. Once that configuration (or a similar configuration is achieved), the annuloplasty ring 100a may be delivered and deployed.

Referring to Fig. 3C, with the annuloplasty ring 100a in the initial assembled state, it may be advanced through catheter 300 (*e.g.,* while it is in a partially or fully collapsed condition. The annuloplasty ring 100a may be advanced by a pusher device in some examples. In some examples, the center opening of the annuloplasty ring 100a may be positioned over the pull wire 220 and/or coil 230, with the pull wire 220 and/or coil 230 acting as a rail over which the annuloplasty ring 100a can be delivered. As noted above, the frame 120a may include anchoring elements, which in Fig. 3C are depicted as barbs 128a, although other anchoring elements may be suitable. If barbs 128a are included, they may be integral with the frame 120a, or formed separately and attached to the frame 120a. During the delivery, if barbs 128a (or another fixation mechanism such as adhesives, hooks, or tines are positioned on the frame 120a and/or the skirt 160a), the annuloplasty ring 100a is preferably advanced over the pull wire 220 and/or coil 230 with the barbs 128a (or other fixation mechanism) facing toward the annulus 22 *(e.g.,* facing the distal or leading end of the catheter 300). Furthermore, in some examples, a capture wire 180 may be coupled to the annuloplasty ring 100a during the advancing. In some examples, the capture wire 180 may be a suture or similar wire that is releasably coupled to the annuloplasty ring 100a. In some example the annuloplasty ring 100a may include a ring 190 or similar structure that the capture wire 180 may be coupled to, although in other embodiments the capture wire 180 may be connected to a strut of the frame of the annuloplasty ring 100a. In some examples, the capture wire 180 may be a suture that is looped through ring 190, with both ends of the suture accessible outside the patient. While the capture wire 180 is coupled to the annuloplasty ring 100a, it may be retrievable back into the catheter 300 by pulling on the capture wire 180. When it is desired to decouple the annuloplasty ring 100a from the capture wire 180, one end of the capture wire may be pulled, allowing the other end to travel toward the ring 190, through the ring 190, and then back through the catheter 300 to fully decouple the capture wire 180 from the annuloplasty ring 100a.

Whether or not capture wire 180 is included, during delivery the pusher device may be advanced distally to push the annuloplasty ring 100a out of the open distal end of the catheter 300, and over the expanding arms 210. As described above, the expanding arms 210 may form a general conical and/or ramped surface over which the annuloplasty ring 100a may be forced. As the annuloplasty ring 100a slides over the expanding arms 210, due at least in part to the flexible nature of the frame 120a, the annuloplasty ring 100a will generally conform to the shape of the expanding arms 210, which matches the shape of the annulus 22 since the annulus 22 has been reshaped by the expanding arms 210. The annuloplasty ring 100a may be further pushed down the ramp formed by the expanding arms 210 until the barbs 128a (or other fixation mechanism(s)) contact and fix into the tissue of the annulus 22, as shown in Fig. 3C. At this point, the sealing skirt 160a (or a portion thereof) is sandwiched between the frame 120a and the annulus 22, and the barbs 128a (or other fixation mechanisms) provide for an initial fixation so that the annuloplasty ring 100a stays coupled to and in contact with the annulus 22. At this point, the capture wire 180 (if included) may be withdrawn, disconnecting the annuloplasty ring 100a from the catheter 300. Then, any applied force on the pull wire 220 may be released and the expanding arms 210 may be re-collapsed into the catheter 300, for example by advancing the catheter 300 distally to force the expanding arms 210 to re-collapse. Then, the catheter 300 may be withdrawn with the sizing device 200 (which may also be referred to as a shaping device) inside the catheter 300, completing this stage of the procedure.

As should be understood from the above, after this stage of the process is completed, the patient's heart may have the configuration of the top view of Fig. 2F, and the annuloplasty ring 100a may have the configuration in the top view of Fig. 2E. Further active intervention by the user is no longer needed at this point. Rather, tissue ingrowth will naturally occur to strongly fix the annuloplasty ring 100a to the patient's annulus 22 over the following days and weeks. The connectors 140a will similarly naturally begin to degrade without further active intervention, but preferably not until significant tissue ingrowth has occurred. As noted above, the connectors 140a may be selected to degrade only after a desired time has passed *(e.g.,* three months), and in some examples different connectors 140a may be used to provide for staggered degradation of the different connectors 140a over time. As the connectors 140a degrade and the individual cells start to transition as shown in Fig. 2D, the annuloplasty ring 100a starts to transition to the bottom configuration of annuloplasty ring 100b shown in Fig. 2E, drawing the annulus 22 to a smaller diameter as shown in the transition of Fig. 2F, ultimately enhancing coaptation and reducing regurgitation across the native tricuspid valve 20b.

It should be understood that the shaping device 200 shown and described above may be particularly suited to situations in which the annuloplasty ring 100a has a mismatch in shape compared to the annulus being treated. If the annuloplasty ring 100a is not designed specifically for a particular patient, given the large patient-to-patient variety in anatomy (particularly at a diseased tricuspid valve), the annuloplasty ring 100a will typically not match the patient's anatomy. As should be understood from the above, this mismatch may be overcome via the use of the shaping device 200. However, in other embodiments, the annuloplasty ring 100a may be provided as a patient-specific device. For example, prior to performing the annuloplasty procedure, the patient's anatomy may be imaged, for example using CT imaging or using another imaging modality. The shape of the particular patient's anatomy (*e.g*., the shape of the tricuspid valve annulus) may be determined from the imaging, and the annuloplasty ring 100a, and particularly the frame 120a, may be shape-set to match the patient anatomy. If the annuloplasty ring 100a is personalized to the patient, in some examples the shaping device 200 may be omitted, such that the annuloplasty ring 100a may be directly pressed into contact with the annulus 22, maintaining good contact to allow tissue ingrowth due to the matching shape between the annuloplasty ring 100a and the annulus 22.

Further, although addressed above, it is reiterated here that the concepts relating to annuloplasty ring 100a described here may be used with annuloplasty rings to treat the mitral valve, aortic valve, or pulmonary valve, even if the annuloplasty ring 100a is particularly well suited to treat the tricuspid valve.

## Claims

1. An annuloplasty ring system comprising:
an annuloplasty ring (100a), comprising:
a frame (120a) having a plurality of cells, each of the plurality of cells having a diamond shape with a top apex, a bottom apex and two side apices, the plurality of cells forming a circumferential ring;
a skirt (160a) coupled to a bottom surface of the frame; and
a plurality of biodegradable connectors (140a), each of the plurality of biodegradable connectors connecting the top apex of a corresponding one of the plurality of cells to the bottom apex of the corresponding one of the plurality of cells,
wherein the frame is formed of a shape-memory material and is shape-set to a final condition in which the frame has a final diameter (Db), the plurality of biodegradable connectors maintaining the frame in an initial condition having an initial diameter (Da), the initial diameter being larger than the final diameter.

2. The annuloplasty ring system of claim 1, wherein one of the side apices of each of the plurality of cells is connected to one of the side apices of an adjacent one of the plurality of cells.

3. The annuloplasty ring system of claim 1, wherein the corresponding one of the plurality of cells includes a top aperture (124a) formed in the top apex and a bottom aperture (124a) formed in the bottom apex, a corresponding one of the plurality of biodegradable connectors (140a) having a first end knotted at the top aperture and a second end knotted at the bottom aperture.

4. The annuloplasty ring system of claim 1, wherein the plurality of biodegradable connectors (140a) are biodegradable sutures.

5. The annuloplasty ring system of claim 4, wherein the frame (120a) is configured to transition from the initial condition to the final condition upon degradation of the biodegradable sutures.

6. The annuloplasty ring system of claim 5, wherein the biodegradable sutures (140a) are configured to maintain the frame (120a) in the initial condition for more than about one month following implantation of the annuloplasty ring into a patient.

7. The annuloplasty ring system of claim 1, wherein each of the plurality of cells has an initial height (Ha) and an initial width (Wa) in the initial condition of the frame (120a), and a final height (Hb) and a final width (Wb) in the final condition of the frame (120b), the initial height being smaller than the final height, the final width being smaller than the initial width.

8. The annuloplasty ring system of claim 1, wherein the skirt (160a) includes a first skirt portion and a second skirt portion, the first skirt portion being coupled to the bottom surface of the frame (120a), the second skirt portion being coupled to a top surface of the frame so that the frame is sandwiched between the first skirt portion and the second skirt portion.

9. The annuloplasty ring system of claim 1, wherein the frame (120a) includes a plurality of hooks, tines, or barbs extending away from the bottom surface of the frame.

10. The annuloplasty ring system of claim 1, further comprising adhesives on one or both of (i) the bottom surface of the frame (120a) and (ii) the skirt (160a).

11. The annuloplasty ring system of claim 1, further comprising:
a sizing device (200) comprising:
a plurality of expandable arms (210), each of the plurality of expandable arms having a first end a free end remote from the first end, the first end of each of the plurality of expandable arms being coupled together at a first connection point (211).

12. The annuloplasty ring system of claim 11, wherein the sizing device (200) includes a plurality of actuator arms (216), each of the actuator arms having a first end coupled to a corresponding one of the plurality of expandable arms, the second end of each of the plurality of actuator arms being coupled together at a second connection point (218).

13. The annuloplasty ring system of claim 12, wherein the sizing device (200) includes a pull wire (220) extending through a coil member (230), the pull wire being coupled to the second connection point (218) such that pulling the pull wire expands the plurality of actuator arms (216) radially outwardly, and expansion of the plurality of actuator arms radially outwardly causes the plurality of expandable arms (210) to expand radially outwardly.

## Patentansprüche

1. Annuloplastieringsystem, umfassend:
einen Annuloplastiering (100a), umfassend:
einen Rahmen (120a), der eine Vielzahl von Zellen aufweist, wobei jede der Vielzahl von Zellen eine Rautenform mit einer oberen Spitze, einer unteren Spitze und zwei seitlichen Spitzen aufweist, wobei die Vielzahl von Zellen einen umlaufenden Ring bilden;
einen Rock (160a), der mit einer unteren Oberfläche des Rahmens gekoppelt ist; und
eine Vielzahl von biologisch abbaubaren Verbindungselementen (140a), wobei jedes der Vielzahl von biologisch abbaubaren Verbindungselementen den oberen Scheitelpunkt einer entsprechenden der Vielzahl von Zellen mit dem unteren Scheitelpunkt der entsprechenden der Vielzahl von Zellen verbindet,
wobei der Rahmen aus einem Formgedächtnismaterial gebildet ist und auf einen Endzustand formfixiert ist, in dem der Rahmen einen Enddurchmesser (Db) aufweist, wobei die Vielzahl von biologisch abbaubaren Verbindungselementen den Rahmen in einem Anfangszustand mit einem Anfangsdurchmesser (Da) hält, wobei der Anfangsdurchmesser größer als der Enddurchmesser ist.

2. Annuloplastieringsystem nach Anspruch 1, wobei eine der seitlichen Spitzen jeder der Vielzahl von Zellen mit einer der seitlichen Spitzen einer benachbarten der Vielzahl von Zellen verbunden ist.

3. Annuloplastieringsystem nach Anspruch 1, wobei die entsprechende der Vielzahl von Zellen eine obere Öffnung (124a), die in der oberen Spitze gebildet ist, und eine untere Öffnung (124a) einschließt, die in der unteren Spitze gebildet ist, wobei ein entsprechendes der Vielzahl von biologisch abbaubaren Verbindungselementen (140a) ein erstes Ende, das an der oberen Öffnung verknotet ist, und ein zweites Ende aufweist, das an der unteren Öffnung verknotet ist.

4. Annuloplastieringsystem nach Anspruch 1, wobei die Vielzahl von biologisch abbaubaren Verbindungselementen (140a) biologisch abbaubare Nähte sind.

5. Annuloplastieringsystem nach Anspruch 4, wobei der Rahmen (120a) so ausgebildet ist, dass er bei Abbau der biologisch abbaubaren Nähte von dem Anfangszustand in den Endzustand übergeht.

6. Annuloplastieringsystem nach Anspruch 5, wobei die biologisch abbaubaren Nähte (140a) so ausgebildet sind, dass sie den Rahmen (120a) für mehr als etwa einen Monat nach der Implantation des Annuloplastierings in einen Patienten in seinem Anfangszustand halten.

7. Annuloplastieringsystem nach Anspruch 1, wobei jede der Vielzahl von Zellen eine Anfangshöhe (Ha) und eine Anfangsbreite (Wa) in dem Anfangszustand des Rahmens (120a) sowie eine Endhöhe (Hb) und eine Endbreite (Wb) in dem Endzustand des Rahmens (120b) aufweist, wobei die Anfangshöhe kleiner als die Endhöhe ist, wobei die Endbreite kleiner als die Anfangsbreite ist.

8. Annuloplastieringsystem nach Anspruch 1, wobei der Rock (160a) einen ersten Rockabschnitt und einen zweiten Rockabschnitt einschließt, wobei der erste Rockabschnitt an die untere Oberfläche des Rahmens (120a) gekoppelt ist, wobei der zweite Rockabschnitt an eine obere Oberfläche des Rahmens gekoppelt ist, sodass der Rahmen zwischen den ersten Rockabschnitt und den zweiten Rockabschnitt eingeschoben ist.

9. Annuloplastieringsystem nach Anspruch 1, wobei der Rahmen (120a) eine Vielzahl von Haken, Zinken oder Widerhaken, die sich von der unteren Oberfläche des Rahmens weg erstrecken, einschließt.

10. Annuloplastieringsystem nach Anspruch 1, weiter umfassend Klebstoffe auf einer oder beiden von (i) der unteren Oberfläche des Rahmens (120a) und (ii) des Rocks (160a).

11. Annuloplastieringsystem nach Anspruch 1, weiter umfassend:
eine Größenbestimmungsvorrichtung (200), umfassend:
eine Vielzahl von aufweitbaren Armen (210), wobei jeder der Vielzahl von aufweitbaren Armen ein erstes Ende und ein von dem ersten Ende entferntes freies Ende aufweist, wobei das erste Ende jedes der Vielzahl von aufweitbaren Armen an einem ersten Verbindungspunkt (211) miteinander gekoppelt ist.

12. Annuloplastieringsystem nach Anspruch 11, wobei die Größenbestimmungsvorrichtung (200) eine Vielzahl von Betätigungsarmen (216) einschließt, wobei jeder der Betätigungsarme ein erstes Ende aufweist, das mit einem entsprechenden der Vielzahl von aufweitbaren Armen gekoppelt ist, wobei das zweite Ende jedes der Vielzahl von Betätigungsarmen an einem zweiten Verbindungspunkt (218) miteinander gekoppelt ist.

13. Annuloplastieringsystem nach Anspruch 12, wobei die Größenbestimmungsvorrichtung (200) einen Zugdraht (220) einschließt, der sich durch ein Spulenelement (230) erstreckt, wobei der Zugdraht derart mit dem zweiten Verbindungspunkt (218) gekoppelt ist, dass durch das Ziehen des Zugdrahts die Vielzahl von Betätigungsarmen (216) radial nach außen aufgeweitet wird und das Aufweiten der Vielzahl von Betätigungsarmen radial nach außen veranlasst, dass die Vielzahl von aufweitbaren Armen (210) sich radial nach außen aufweitet.

## Revendications

1. Système d'anneau d'annuloplastie comprenant :
un anneau d'annuloplastie (100a), comprenant :
un cadre (120a) présentant une pluralité de cellules, chacune de la pluralité de cellules présentant une forme de diamant avec un sommet supérieur, un sommet inférieur et deux sommets latéraux, la pluralité de cellules formant un anneau circonférentiel ;
une jupe (160a) couplée à une surface inférieure du cadre ; et
une pluralité de connecteurs biodégradables (140a), chacun de la pluralité de connecteurs biodégradables reliant le sommet supérieur d'une cellule correspondante de la pluralité de cellules au sommet inférieur de l'une cellule correspondante de la pluralité de cellules,
dans lequel le cadre est formé d'un matériau à mémoire de forme et est mis en forme dans un état final dans lequel le cadre a un diamètre final (Db), la pluralité de connecteurs biodégradables maintenant le cadre dans un état initial ayant un diamètre initial (Da), le diamètre initial étant plus grand que le diamètre final.

2. Système d'anneau d'annuloplastie selon la revendication 1, dans lequel l'un des sommets latéraux de chacune de la pluralité de cellules est relié à l'un des sommets latéraux d'une adjacente de la pluralité de cellules.

3. Système d'anneau d'annuloplastie selon la revendication 1, dans lequel l'une cellule correspondante de la pluralité de cellules inclut une ouverture supérieure (124a) formée dans le sommet supérieur et une ouverture inférieure (124a) formée dans le sommet inférieur, un connecteur correspondant de la pluralité de connecteurs biodégradables (140a) présentant une première extrémité nouée au niveau de l'ouverture supérieure et une deuxième extrémité nouée au niveau de l'ouverture inférieure.

4. Système d'anneau d'annuloplastie selon la revendication 1, dans lequel la pluralité de connecteurs biodégradables (140a) sont des sutures biodégradables.

5. Système d'anneau d'annuloplastie selon la revendication 4, dans lequel le cadre (120a) est configuré pour passer de l'état initial à l'état final lors de la dégradation des sutures biodégradables.

6. Système d'anneau d'annuloplastie selon la revendication 5, dans lequel les sutures biodégradables (140a) sont configurées pour maintenir le cadre (120a) dans l'état initial pendant plus d'un mois environ après l'implantation de l'anneau d'annuloplastie chez un patient.

7. Système d'anneau d'annuloplastie selon la revendication 1, dans lequel chacune de la pluralité de cellules présente une hauteur initiale (Ha) et une largeur initiale (Wa) dans l'état initial du cadre (120a), et une hauteur finale (Hb) et une largeur finale (Wb) dans l'état final du cadre (120b), la hauteur initiale étant plus petite que la hauteur finale, la largeur finale étant plus petite que la largeur initiale.

8. Système d'anneau d'annuloplastie selon la revendication 1, dans lequel la jupe (160a) inclut une première partie de jupe et une deuxième partie de jupe, la première partie de jupe étant couplée à la surface inférieure du cadre (120a), la deuxième partie de jupe étant couplée à une surface supérieure du cadre de sorte que le cadre soit pris en sandwich entre la première partie de jupe et la deuxième partie de jupe.

9. Système d'anneau d'annuloplastie selon la revendication 1, dans lequel le cadre (120a) inclut une pluralité de crochets, de dents ou de barbes s'étendant à l'écart de la surface inférieure du cadre.

10. Système d'anneau d'annuloplastie selon la revendication 1, comprenant en outre des adhésifs sur l'une de (i) la surface inférieure du cadre (120a) et (ii) la jupe (160a) ou les deux à la fois.

11. Système d'anneau d'annuloplastie selon la revendication 1, comprenant en outre :
un dispositif de dimensionnement (200) comprenant :
une pluralité de bras extensibles (210), chacun de la pluralité de bras extensibles présentant une première extrémité une extrémité libre éloignée de la première extrémité, la première extrémité de chacun de la pluralité de bras extensibles étant couplée ensemble au niveau d'un premier point de connexion (211).

12. Système d'anneau d'annuloplastie selon la revendication 11, dans lequel le dispositif de dimensionnement (200) inclut une pluralité de bras d'actionneur (216), chacun des bras d'actionneur présentant une première extrémité couplée à l'un bras correspondant de la pluralité de bras extensibles, la deuxième extrémité de chacun de la pluralité de bras d'actionneur étant couplée ensemble au niveau d'un deuxième point de connexion (218).

13. Système d'anneau d'annuloplastie selon la revendication 12, dans lequel le dispositif de dimensionnement (200) inclut un fil de traction (220) s'étendant à travers un élément de bobine (230), le fil de traction étant couplé au deuxième point de connexion (218) de sorte que la traction du fil de traction étend la pluralité de bras d'actionneur (216) radialement vers l'extérieur, et l'extension de la pluralité de bras d'actionneur radialement vers l'extérieur amène la pluralité de bras extensibles (210) à s'étendre radialement vers l'extérieur.
